Europäisches Patentamt

European Patent Office

Office européen des brevets

(11) Publication number: **0 186 476**
**A2**

# EUROPEAN PATENT APPLICATION

(21) Application number: 85309363.1

(22) Date of filing: 20.12.85

(51) Int. Cl.⁴: **G 01 N 33/48**
**G 01 N 33/68**

(30) Priority: 21.12.84 US 684958

(43) Date of publication of application:
02.07.86 Bulletin 86/27

(84) Designated Contracting States:
AT BE CH DE FR GB IT LI LU NL SE

(71) Applicant: HEMOCHEK CORPORATION
912 NW 13th Street
Gainesville Florida 32601(US)

(72) Inventor: Clemmons, Roger M.
1911 N.W. 20th Way
Gainesville Florida 32605(US)

(72) Inventor: Goldman, Richard A.
3451 N.W. 35th Place
Gainesville Florida 32605(US)

(74) Representative: Hildyard, Edward Martin et al,
Frank B. Dehn & Co. Imperial House 15-19 Kingsway
London WC2B 6UZ(GB)

(54) Diagnostic kit and method for assaying blood components.

(57) A method and diagnostic kit are disclosed for screening plasma for von Willebrand's disease (deficiency factor VIIIR:WF activity) and for abnormalities in other blood components in human and animal blood. The disclosed dianostic kit permits rapid, relatively inexpensive differentiation between individuals having Type I and Type II von Willebrand's disease, respectively, can be used in a semi-quantitative determination of the quantity of factor VIIIR:WF activity in blood products, and can also be used to detect Bernard-Soulier syndrome. Cryopreserved, fixed, fresh-washed and artificial platelets, and ristocetin, ristomycin, polybrene, and a derivative of snake venom, can be used in the diagnostic kit.

"<u>Diagnostic kit and method for assaying</u>
<u>blood components</u>"

## BACKGROUND OF THE INVENTION

The present invention relates to both a method and a diagnostic kit for screening human and animal plasmas for various blood abnormalities, including deficiencies in factor VIIIR:WF (von Willebrand's factor) activity (von Willebrand's disease) and Bernard-Soulier syndrome. The present invention can also be used to differentiate between individuals suffering from Type I and Type II von Willebrand's disease, respectively.

One of the important blood components necessary for adequate primary hemostasis is factor VIIIR:WF, or von Willebrand's factor (vWF), also known as "platelet agglutinating factor" (PAF or vWF/PAF), as "ristocetin cofactor," and as "bovine (or porcine) antihemophilic factor." This plasma protein is quantitatively or qualitatively abnormal in von Willebrand's disease (vascular hemophilia), which occurs in man and animals (e.g., dog and pig).

In Type I von Willebrand's disease, the levels of factor VIIIR:WF are reduced, or the factor may be absent. In Type II von Willebrand's disease, the levels of ristocetin-induced cofactor activity are reduced, but the levels of factor VIIIR:WF activity induced by certain snake venoms are normal (Howard et al., "The agglutination of human platelets by botrocetin: Evidence that botrocetin and ristocetin act at different sites of the factor VIII molecule and platelet membrane," Brit. J. Haematol. 57:25-35, 1984, the contents of which are hereby incorporated by reference). Thus, while the vWF protein is present in normal quantities in individuals who have Type II von Willebrand's disease, the protein is functionally abnormal.

This plasma factor is also altered in certain disease states, and is thought to be important in the recruitment of platelets to sites of developing atherosclerosis. Accordingly, in treating patients with stored blood components, e.g., to control hemorrhaging or to replace lost blood, it is important to be able to determine quickly the amount of factor VIIIR:WF activity in the stored blood component.

Clinically, there are three methods of measuring factor VIIIR:WF activity in plasma:

(1) platelet agglutination to various cofactors in an aggregometer, which is essentially a modified spectrophotometer;

(2) the macroscopic platelet agglutination time in a test tube; and

(3) immunophoresis of plasma for factor VIIIR:WF-related antigen (factor VIIIR:Ag), a large protein which contains factor VIIIR:WF.

Methods (1) and (2) involve the use of (a) citrated plasma or other body fluid to be tested for factor VIIIR:WF activity; (b) a suspension of platelets for the determination of the rate of platelet agglutination; and (c) a cofactor (such as ristocetin, snake venom, and bovine or porcine antihemophilic factor) to induce platelet agglutination. Method (3) utilizes citrated plasma and specific antibodies to factor VIIIR:Ag.

Platelet agglutination to ristocetin in an aggregometer, using fresh platelet-rich plasma or fixed platelets resuspended in platelet-poor plasma, has been described, for example, by Howard et al., "Ristocetin - a new tool in the investigation of platelet

- 3 -

0186476

aggregation," Thromb. Diath. Haemorrh. 26:362-369, 1971; Weiss et al., "Quantitative assay of plasma factor deficient in von Willebrand's disease that is necessary for platelet aggregation," J. Clin. Invest. 52:2708-2716, 1973; Macfarlane et al., "A method for assaying von Willebrand factor (ristocetin cofactor)," Thromb. Diath. Haemorrh. 34:306-308, 1975; and Allain et al., "Platelets fixed with paraformaldehyde: A new reagent for assay of von Willebrand factor and platelet aggregating factor," J. Lab. Clin. Med. 85:318-328, 1975. Fresh-washed platelets, fixed platelets and cryopreserved platelets (i.e., platelets previously frozen in solution or lyophilized) can be used in this procedure as the agglutinating substrate (see, e.g., Odink, "Platelet preservation. II. The response of human platelet suspensions of hypotonic stress," Thomb. Haemostas. 36:182-191, 1976).

Ristocetin, certain snake venoms, polybrene, and bovine or porcine antihemophilic factor may be used for the cofactor which induces platelet agglutination in the presence of normal factor VIIIR:WF (see, e.g., Leis et al., "Ristocetin-induced aggregation of canine platelets," Thromb. Res. 19:309-316, 1980; Brinkhous et al., "Use of venom coagglutinin and lyophilized platelets in testing for platelet-aggregating von Willebrand factor," Blood 55:517-520 1980; Johnson et al., "Species specificity in von Willebrand factor-supported platelet agglutination," Annals NY Acad. Sci. 81:227-235, 1981; Forbes et al., "Aggregation of human platelets by purified porcine and bovine antihaemophilic factor," Nature 241:149-150, 1973). The respective contents of the documents cited in the preceding sentence are hereby incorporated by reference. Rather than combining with factor VIIIR:WF in plasma to effect agglutination, as do the other cofactors mentioned above, bovine and porcine antihemophilic factor combines with a platelet-associated constituent to cause agglutination. Accordingly, bovine and procine antihemophilic factor can be isolated and used as an agglutination-inducing cofactor for platelets other than bovine or porcine in plasma which is not taken from cow or pig.

The degree of agglutination, and the nature of the agglutination reaction, varies among the various cofactors. Ristocetin is the most frequently employed agglutinating agent, but has the disadvantage of initiating the precipitation of plasma proteins. Such precipitation can lead to incorrect results, in both the aggregometery and the tube agglutination methods. In addition, ristocetin does not induce agglutination of platelets in the presence of canine plasma and, even with the addition of high concentrations of albumin (Rosborough et al., "Measurement of canine von Willebrand factor using ristocetin and polybrene," _J. Lab. Clin. Med._ 96:47-56, 1980, the contents of which are hereby incorporated by reference), the agglutination reaction is very limited, rendering differentiation between the two types of von Willebrand's disease impractical in the dog. The aggregometery method also has the disadvantages of being costly and requiring technically advanced equipment, thereby reducing the effectiveness and ease of performing the determination. In addition, platelet aggregometery, where results are based upon the degree of agglutination induced by the cofactor over a specific time, may be influenced by the presence of turbid plasma samples, rendering the test results unreliable.

In method (2) mentioned above, the time required for macroscopic agglutination to occur in a test tube determines the time of initial formation of visible aggregates of platelets, the initial-formation time being the key parameter of the test (see Sarjie et al., "Nature of von Willebrand factor: A new assay and a specific inhibitor," _Proc. Nat. Acad. Sci. U.S.A._, 71:2937-2941, 1974; Brinkhous et al., "Assay of von Willebrand factor in von Willebrand's disease and hemophilia: Use of a macroscopic platelet aggregation test," _Thromb. Res._ 6:267-272, 1975). Although method (2) is less time consuming and requires no expensive equipment, it is often difficult to determine the exact time that aggregates first become visible, making results based on the initial-formation time less reliable. Also, ristocetin-induced precipitation of plasma proteins may be mistaken for platelet aggregates.

Although certain snake venoms overcome the problem of protein-precipitation encountered with ristocetin, snake venoms cannot separate Type II von Willebrand individuals, who test as normal. Another drawback to the aggregometery and macroscopic tube methods is the requirements in both for relatively large quantities of platelets, plasma, and cofactor, making them expensive to perform.

The determination of factor VIIIR:Ag by method (3) (see, e.g., Zimmerman et al., "Immunologic differentiation of classic hemophilia [factor VIII deficiency] and von Willebrand's disease," J. Clin. Invest. 50:244-254, 1971) is used only in conjunction with one of the other two methods, since immunophoresis is time-consuming, requires expensive equipment, and only quantitates the plasma level of factor VIIIR:Ag, i.e., method (3) does not determine whether the antigen is functional. Accordingly, immunophoresis alone can only identify Type I von Willebrand patients. In order to differentiate between Type I and Type II von Willebrand's disease, therefore, it has been necessary to ascertain the agglutinating effects of ristocetin and snake venom, respectively, or both to test for agglutination with ristocetin and also to determine immunologically the levels of factor VIIIR:Ag.

Another important blood abnormality is Bernard-Soulier syndrome. In human patients with Bernard-Soulier syndrome, factor VIIIR:WF is generally normal but the blood platelets lack glycocalicin, a platelet-membrane glycoprotein necessary to factor VIIIR:WF-induced agglutination, and thus do not agglutinate normally. See Shulman & Karpatkin, "Crossed immunoelectrophoresis of human platelet membranes: Diminished major antigen in Glanzmann's thrombasthenia and Bernard-Soulier syndrome," J. Biol. Chem. 255: 4320-4327 (1980). As with von Willebrand's disease, there is an unmet need for a way to screen blood samples for evidence of Bernard-Soulier syndrome in a variety of situations, including in hospital emergency rooms and in blood banks.

0186476

## SUMMARY OF THE INVENTION

In accordance with one aspect of the present invention, a diagnostic kit for analyzing blood plasma of a particular species comprises

(a) first means for visualizing finely-divided punctate bodies dispersed in a predetermined amount of liquid, the first means comprising means for comparing, by direct ocular inspection, at least two samples, each sample comprising a predetermined amount of liquid and at least one sample containing the finely-divided punctate bodies;

(b) second means for introducing into the first means a predetermined amount of a mixture comprising blood plasma and particles capable of agglutination in the blood plasma in the presence of a cofactor; and

(c) third means for introducing into the first means a predetermined amount of a solution comprising a cofactor for agglutination of the particles.

In one preferred embodiment, the particles in the above-described diagnostic kit comprise blood platelets. In another preferred embodiment, the aforementioned third menas comprises (i) means for introducing into the first means a solution comprising a first agglutination cofactor and (ii) means for introducing into the first means a solution comprising a second agglutination cofactor.

According to a particularly preferred embodiment, a diagnostic kit for analysing blood plasma for factor VIII R:WF comprises

(a) agglutination slides or other means for displaying test and control samples in a manner permitting ocular comparison;

(b) lyophilised blood platelets, normal plasma, and at least one dilution of normal plasma, or means for reconstituting said normal and diluted plasma; and

(c)    cofactors for agglutination of said platelets, said cofactors comprising (i) a cofactor for agglutination of said platlets in plasma containing normal factor VIIIR:WF, and (ii) a cofactor for agglutination of said platelets in plasma containing abnormal factor VIIIR:WF (i.e. as present in Type II von Willebrand's disease).

The diagnostic kit of our invention is simple and comparatively inexpensive to use, and as described hereinafter, can be expanded to permit the semi-quantitative assaying of various blood components including plasma factor VIIIR:WF and platelet protein glycogalicin, in blood samples.

Moreover the kit can be formulated to provide means for rapidly differentiating between individuals who suffer from Type I von Willebrand's disease from those suffering from Type II von Willebrand's disease; the diagnosis can also be relatively unaffected by plasma sample turbidity and it eliminates time to initial formation of platelet aggregates as a factor introducing error into the final determination.

Our diagnostic kit and method can also be formulated to provide means for detecting Bernard-Soulier syndrome in humans and animals.

In accordance with another aspect of the present invention, there has been provided a method for analyzing blood plasma, comprising the steps of

(a) preparing a plurality of samples, each sample having a known volume and comprising, respectively, a predetermined concentration of blood plasma of a first species and particles capable of agglutination in the blood plasma in the presence of a cofactor, the plurality of samples comprising at least (i) a first sample comprising the particles and a known concentration of normal plasma of the first species and (ii) a second sample comprising a known concentration of the particles and blood plasma from a particular individual of the first species;

(b) adding to at least some of the samples of the plurality of samples a predetermined amount of a cofactor for agglutination of the particles in blood plasma of the first species; and then,

(c) after an interval of a duration such that agglutination of the particles by the cofactor is substantially complete, identifying by direct ocular inspection those samples of the plurality of samples wherein punctate bodies are dispersed.

Preferably, the particles used in the method of the present invention comprise blood platelets, and step (b) comprises (i) adding a first cofactor for agglutination of the blood platelets to a predetermined number of the samples of the plurality of samples, and (ii) adding a second cofactor for agglutination of the blood platelets to at least some of the remaining samples of the plurality.

Other objects, features, and advantages of the present invention will become apparent from the following detailed description. It should be understood, however, that the detailed description and specific examples, while indicating preferred embodiments of the invention, are given by way of illustration only, since various changes modifications within the spirit and scope of the invention will become apparent to those skilled in the art from this detailed description.

Moreover the following description relates specifically to analysis of blood plasma for factor VIIIR:WF, but the diagnostic kit of our invention can readily be formulated for analysing other agglutination factors in plasma.

## BRIEF DESCRIPTION OF THE DRAWINGS

Figure 1 is a perspective view of a diagnostic kit within the present invention.

Figure 2 presents planar and transverse perspectives, respectively, of agglutination slides that can be used, in accordance with the present invention, as means for visualizing punctate platelet aggregates dispersed in a sample containing blood plasma.

Figures 3-5 show various items that can be used a components of a diagnostic kit within the present invention.

## DETAILED DESCRIPTION OF THE PREFERRED EMBODIMENT

The diagnostic kit of the present invention preferably contains lyophilized blood platelets, dilutions of normal plasma and, as cofactors, ristocetin and a derivative of snake venom (e.g., from Bothrops jararaca). In addition, the kit can contain agglutination slides, patient-plasma pipet tips, and a pipet bulb. As indicated above, the present invention provides a bioassay for factor VIIIR:WF in human and animal plasma, which bioassay compares the degree of agglutination (size and number of platelet aggregates) for platelets mixed with test plasma and the agglutination cofactor(s) with the degree of agglutination of platelets mixed with normal plasma (preferably, with various dilutions of pooled-normal plasma) and the same cofactor(s).

Reference has already been made to the use of "pooled-normal" blood plasma in the present invention. Plasma samples from several conspecific individuals, preferably six or more, are combined to produce "pooled-normal" plasma. Since the composite characteristics of pooled-normal plasma are less likely to reflect any biochemical idiosyncrasies of the individuals who contributed to the pooled sample, pooled-normal plasma is preferred, over plasma from a single "normal" individual, as the control plasma ("normal plasma") against which plasma to be tested for factor VIIIR:WF deficiency ("test plasma") is compared in accordance with the present invention.

Whether the control plasma used in the present invention is pooled-normal or not, it may be desirable in specific instances to determine whether a control sample or a test sample contains antibodies against platelets mixed in that sample, as described in greater detail below, or against factor VIIIR:Ag itself. Although their presence is considered an unlikely possibility, anti-platelet antibodies would increase, and anti-factor VIIIR:Ag antibodies decrease, the apparent level of factor VIIIR:WF activity, producing a false positive and a false negative determination, respectively. One control against the presence of anti-platelet antibodies would be to test for the formation of aggregates in the absence of any cofactor. The presence of anti-factor VIIIR:Ag antibodies could be detected by mixing test plasma with control plasma in a ratio of about 1:3 and then comparing agglutination activity of the resultant combined-plasma sample against that of the test plasma and control plasma, respectively. If the activity level in the combined-plasma sample is less than additive, relative to the constituent plasmas, the presence of anti-factor VIIIR:Ag antibodies can be inferred. Once detected, undesired antibodies could be eliminated by conventional techniques, such as column extraction.

An important feature of the present invention is the direct observation, by ocular inspection, of finely divided, punctate aggregates of blood platelets that form in "normal" samples (i.e., those derived from blood plasma displaying a normal complement of procoagulant, cellular clotting and platelet aggregating activities)

- 11 -

0186476

after an agglutination cofactor has been introduced thereto, and after the resulting agglutination reaction has proceeded substantially to completion. More specifically, the present invention entails a contemporaneous visual comparison, without the need for sophisticated spectrophotometric or other apparatus, between at least (a) one sample containing a known concentration of blood platelets, a known concentration of control ("normal") blood plasma, and an agglutination cofactor and (b) another sample containing the same concentration of platelets, the same cofactor, and the same concentration of test plasma.

It has been discovered that, by directly observing the sample (or samples) containing the test plasma in a contemporaneous (preferably side-by-side) comparison with a control sample which comprises the same concentration of normal (preferably, pooled-normal) plasma, one can readily detect a deficiency of factor VIIIR:WF in the test-plasma sample, following the present invention. A factor VIIIR:WF deficiency is evidenced, in accordance with the present invention, by a paucity and/or a reduction in size, relative to the control samples, of punctate aggregates dispersed in the test-plasma sample.

Preferably, the above-mentioned contemporaneous visual comparison involves a plurality of control samples representing a serial dilution (for example, 100%, 50%, and 25%) of the control plasma in a physiologic saline solution. Utilizing various dilutions of normal plasma in a set of control samples allows one to make a semi-quantitative determination of the effective titer of factor VIIIR:WF in test-plasma sample. Direct visual comparison, in accordance with the present invention, of the relative densities and size of aggregates in the test-plasma sample and in the control samples of differing plasma concentrations, respectively, permits a matching of the test sample with the closest corresponding dilution of normal plasma. Thus, if the density and/or size of aggregates in a test-plasma sample is judged to approximate that of the 25% (4:1) dilution of normal plasma in saline, then the effective titer of factor VIIIR:WF in the test plasma is estimated to be about 25% of normal plasma, following the present invention.

It is also preferred that more than one agglutination cofactor be used in the present invention for the purpose of assessing the relative agglutinating capacity of test-plasma samples versus control samples comprising various amounts of normal plasma. For example, separate comparisons are preferably conducted, in accordance with the present invention, using ristocetin and a derivative of snake venom, respectively, as the agglutinating cofactor. Test plasma from individuals suffering from Type I von Willebrand's disease will show a decrease in agglutinating activity when either cofactor as used. In contrast, samples derived from plasma of individuals suffering from Type II von Willebrand's disease will show decreased ristocetin-induced agglutination only, i.e., the level of snake venom-induced agglutination will be comparatively normal.

Although it has been understood in the art that ristocetin does not readily induce agglutination of platelets in canine plasma, it is nevertheless possible to exploit ristocetin-induced agglutination in analyzing canine plasma in accordance with the present invention. More specifically, it has been discovered that agglutination of platelets in canine plasma can be effected, to an extent readily detectable with the present invention, using ristocetin in the presence of about 300 mg of albumin, preferably serum albumin (bovine, human or canine), per ml with ristocetin. Lesser concentrations of albumin do not prevent ristocetin-induced protein precipitation, thereby introducing an error factor. Also, platelet agglutination is hindered at lesser albumin concentrations, introducing a second error factor.

As previously indicated, the present invention employs ocular inspection and direct visual comparison in lieu of costly aggregometers or similar devices of the sort heretofore used in the art. It must be appreciated, however, that direct visualization of plasma samples in accordance with the present invention can be assisted by means for magnifying the apparent size of platelet aggregates, and for enhancing the contrast between aggregates and background, respectively, thereby making easier the comparison between test and control samples. The aforementioned comparison can also be aided by using reproductions of images that correspond to (or simulate) the "normal" densities and sizes of aggregates previously observed in standardized samples having differing concentrations of control plasma. For example, cards upon which have been printed dot patterns that simulate the respective aggregate densities and sizes in a serial dilution of pooled-normal plasma can be advantageously included in a diagnostic kit within the present invention.

Generally speaking, platelets used in the present invention can be mixed with plasma of any species, i.e., there need be no conspecificity between platelets and the plasma sample wherein the platelets are suspended. An exception to this principle obtains for samples comprising porcine plasma and for samples comprising bovine plasma, as well as other ruminants' plasmas. As previously indicated, it is known that pig and cow plasma normally contains antihemophilic factor which does not cause agglutination of porcine and bovine platelets in vivo, but which, when added to preparations comprising platelets and plasma, respectively, from species neither porcine nor ruminant, acts like ristocetin or snake venom cofactor in causing agglutination. Thus, porcine or bovine antihemophilic factor can serve as agglutinating cofactor in the present invention when samples comprising platelets and plasma, respectively, from non-ruminate, non-porcine species are employed. Conversely, when samples comprising porcine or a ruminant's plasma are assayed in accordance with the present invention, it is preferable that only porcine or ruminant platelets, and a cofactor other than porcine or bovine antihemophilic factor, be used.

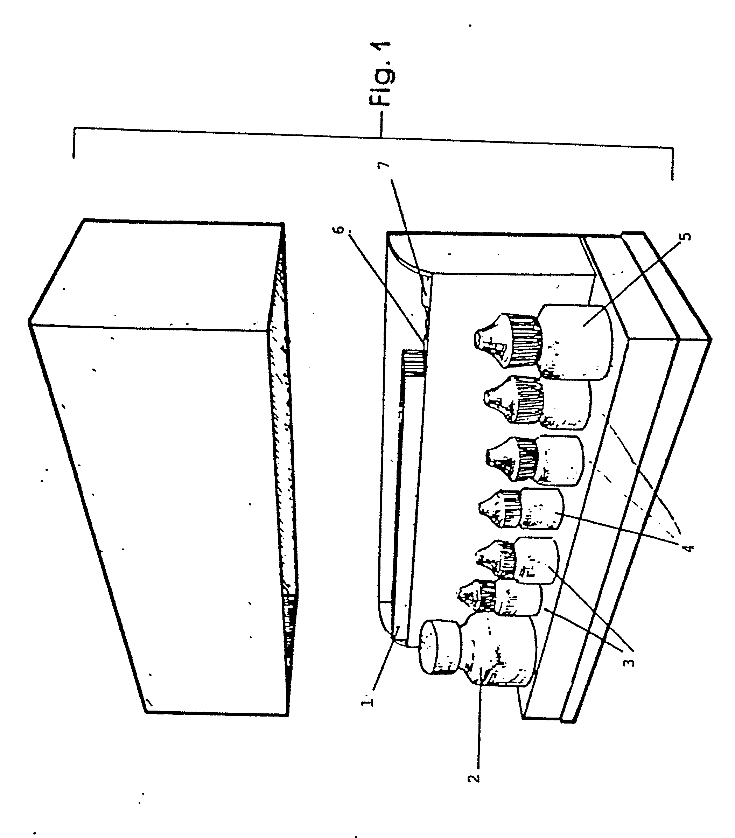

Additional understanding of the present invention can be gained by reference to Figures 1-5, which are offered by way of illustration only.

In Figure 1, which shows a preferred configuration for a diagnostic kit within the present invention, ten agglutination slides (1) are included in a suitable container with a 15-ml injection bottle (2) containing Tris-buffer saline solution (0.15 M at pH 7.4, comprising 0.02% sodium azide) which is used for reconstituting the cofactor(s), plasma and platelets; two 3-ml dropper bottles (3) which contain either lyophilized snake venom (35 /ug per ml) or ristocetin (15 mg/ml with 30% bovine serum albumin); three dropper bottles (4), each containing predetermined amounts of lyophilized, pooled-normal plasma; an 8-ml dropper bottle (5) containing lyophilized (canine) platelets at 400,000 per /ul; ten dropper tips (6) for pipetting test plasma; and a pipet bulb (7).

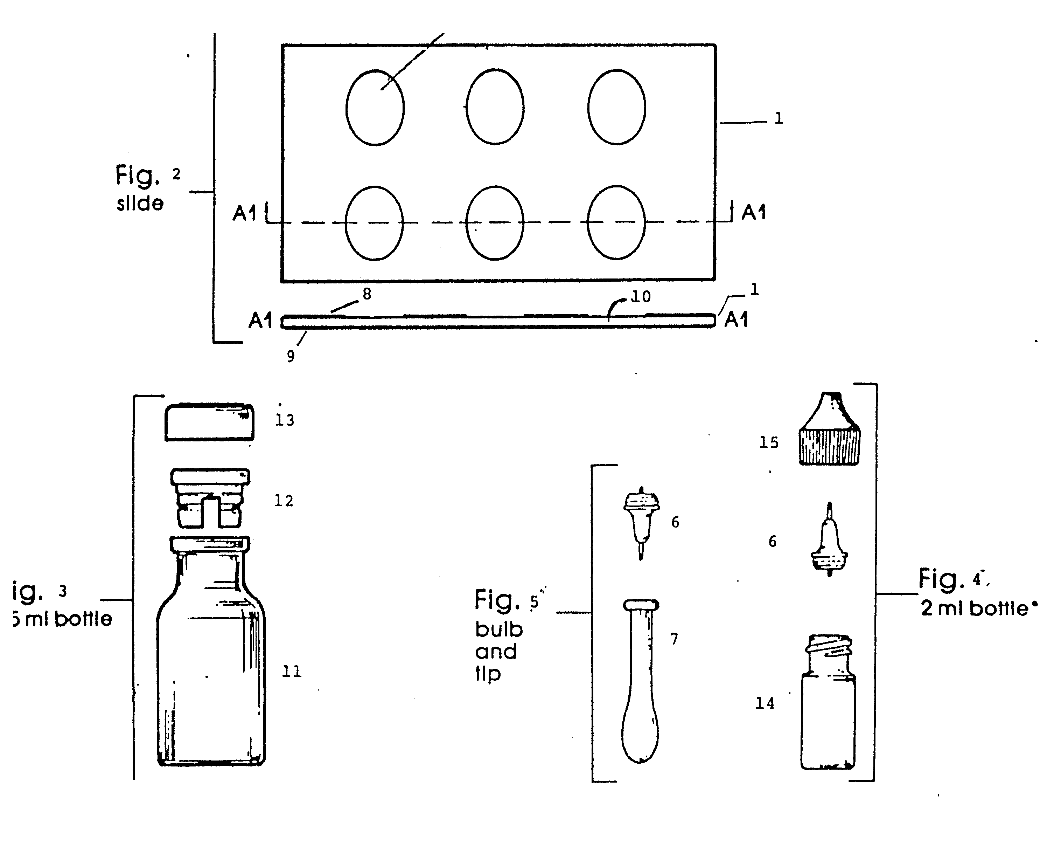

Figure 2 illustrates an agglutination slide which can comprise means for visualizing the punctate platelet aggregates that form in at least some of the plasma samples disposed in each of the slide's wells (8) when the diagnostic kit of the present invention is used. The agglutination slide consists of a wettable, plastic substrate (9) having a silver backing, or mirror surface, and a raised matte front (10). In the matte portion of the slide are provided six ovals wells (8) in which the agglutination reaction (or lack thereof) in each sample can be directly observed against the mirror surface.

Figure 3 shows a 15-ml serum container (11), with rubber stopper (12) and seal (13), which can be used as the injection bottle (2) in the kit shown in Figure 1.

Figure 4 illustrates the configuration of the dropper bottles mentioned above as components (3, 4) in the kit shown in Figure 1. The bottle (14) varies in size from 2 to 8 mls. The pipet tip (6) is standardized, however, so that equal volumes of each reagent can be supplied by mixing 1 drop (at about 20 drops/ml) of the respective reagents in the separate wells of the agglutination slide. The cap (15) is designed to fit the bottle, i.e., to fit over the standardized tip.

Figure 5 depicts the above-mentioned standardized pipet tip (6) with a pipet bulb (7) used to fill the tip with test plasma, which can then be dispensed, one drop at a time, into the wells of the agglutination slide.

In practicing the claimed invention, the reagents of the diagnostic kit are each reconstituted with the buffered saline solution, e.g., by adding to each dropper bottle an amount of saline, preferably prescribed on the bottle itself, to provide the necessary concentration and amount of the respective reagent. A citrated blood sample is obtained from the individual under study and the plasma is separated from blood cells by centrifugation at 1,500 X g for 10 minutes. One drop of reconstituted platelet solution (about 0.05 ml) is then added to each well of the agglutination slide. To two wells, one drop of 100% control plasma is added. To two other wells, one drop of 50% and 25% control plasma, respectively is added. To each of the remaining two wells, one drop of test plasma is added.

The ristocetin cofactor is thereafter added to one of the 100% control samples, to one of the test plasma samples, and to each of the 50% and 25% control plasma sample, respectively. To each of the remaining 100% control and test plasma samples, the snake venom cofactor is added. All the samples are then mixed and layered out over the surface of the respective well, and gently rocked for about three minutes. The samples should be compared within five minutes after addition of the cofactor.

The degree of agglutination to ristocetin cofactor is compared between the 100%, 50%, and 25% control (normal plasma) samples and the first test sample to derive a semi-quantitative determination of the amount of factor VIIIR:WF in the test plasma, as explained above. (Generally, a test plasma sample would be considered "normal" when it displayed factor VIIIR:WF activity in excess of that observed for the 50% control sample.) Then the relative degree of agglutination induced by the snake venom cofactor is ascertained by comparing the 100% control and second test plasma samples. This comparison provides a qualitative determination of whether the factor VIIIR:Ag is functionally normal. Accordingly, for plasma samples from individuals suffering from Type II von Willebrand's disease, the value for factor VIIIR:WF activity in the second test samples (with snake venom cofactor) should be roughly equivalent to activity in the 100% control sample receiving the same cofactor. If a serial dilution series of control samples are used with snake venom cofactor, a semi-quantitative determination can be made, in the manner described above, for the total amount of factor VIIIR:Ag (functionally normal or otherwise) which is present.

The following examples are offered in order to illustrate the present invention more fully, but are not to be construed as limiting the scope thereof.

EXAMPLE 1

Preparation of Platelets

The source of platelets has been found to be of no moment in assaying plasma from human and canine patients in accordance with the present invention. Accordingly, canine platelets can be used in this kit, although platelets from other animal or human sources are also suitable. Formalin-fixed, lyophilized human platelets which can be used in the present invention are marketed by Helena Laboratories (Beaumont, Texas) and by Biodata Corporation (Hatboro, Pennsylvania). To prepare canine platelets for use in the present invention, blood samples were drawn from healthy dogs into acid-citrate-dextrose (ACD), 1 part ACD to 9 parts blood. The citrated samples were centrifuged (2000 X g for 3 minutes at room temperature), or were separated by hemapheresis, to obtain platelet-rich plasma (PRP). To the PRP, 0.25% formalin was added in equal volume, and the mixture allowed to fix overnight at 4°C. The fixed PRP was thereafter centrifuged at 2000 X g for 15 minutes to obtain a fixed-platelet pellet. The platelet pellet was washed (resuspension followed by repeated centrifugation) in 0.15 M phosphate-buffered saline (pH 7.4) a total of four times. Two washings in 0.15 M Tri-buffered saline at pH 7.4 then followed. Finally, the platelet pellet was washed twice in deionized water containing 0.01% sodium azide. After the final wash, the resuspended platelets were allowed to stand for one hour, and the supernatant was removed. The platelet count was adjusted to 400,000 platelets per /ul. The sample was divided into the dropper bottles and lyophilized. Platelets prepared in this manner were found to be stable for over 12 months (and for 30 days following reconstitution) when stored at 4°C .

EXAMPLE 2

Preparation of Plasma Controls:

Blood samples drawn in ACD from healthy individuals of the same species were centrifuged at 2000 X g for 30 minutes at 4°C to obtain platelet-poor plasma. Alternatively, platelet-poor plasma was obtained by hemapheresis. The plasma was then divided into dropper bottles (2 ml for the 100% control sample; 1 ml for the 50% control sample and 0.5 ml for the 25% control sample) and lyophilized. The lyophilized products were each later reconstituted with 2 ml of Tris-buffered saline to produce the desired plasma dilutions.

EXAMPLE 3

Preparation of Cofactors:

The snake venom cofactor was prepared from Bothrops jararaca venom (obtained from Sigma Chemical Company, St. Louis, Missouri). To separate venom agglutination cofactor from other venom constituents, including factors which display thrombin-like activity, see, e.g., Mitrakul, "Effects of Green Pit Viper... venoms on blood coagulation, platelets and the fibrinolytic enzyme systems," Am. J. Chem. Pharm. 60: 654-62 (1973), the snake venom was dissolved in 0.15 M saline containing 84 mM imidizole at pH 7.35 at a concentration of 5 mg/ml and then dialyzed against buffer at 4°C for 24 hours. The platelet agglutinating fraction was obtained by eluting the dialyzed venom through diethylaminoethyl cellulose resin (ion-exchange chromatography) with 0.2 M saline containing 84 mM imidizole (pH 7.35). The eluted fraction was then dialyzed against deionized water for 24 hours at 4°C, diluted to 35 μg per ml, divided into dropper bottles (1.5 ml), and finally lyophilized.

Ristocetin cofactor was made by weighing 37.5 mg of ristocetin and 750 mg of bovine serum albumin into a 3-ml dropper bottle. The contents of the dropper bottle reconstituted with 2.5 ml Tris-buffered saline to achieve a working concentration of 15 mg/ml ristocetin in 30% bovine serum albumin. At this concentration of albumin, ristocetin does not induce plasma protein precipitation, but still supports platelet agglutination in the presence of functional factor VIIIR:Ag.

Other cofactors may be substituted for both ristocetin and snake venom cofactors in the present invention. As a source of snake venom from which a suitable snake venom cofactor can be derived, snakes of the family Crotalidae are preferred. Although Bothrops jararaca venom is particularly preferred, other venoms can be used in the present invention, including venoms of Bothrops alternatus, Bothrops neowiidis, Trimeresurus flavoviridis, A. rhodostoma, Bothrops atrox, Bothrops jararacussu, and Bothrops lansbergii. See U.S. Patent No. 4,287,087, the contents of which are hereby incorporatred by reference.

A purified snake venom cofactor is also manufactured commercially, under the product name BROTROCETIN, by Pentapharm AG of Basel, Switzerland. Ristocetin cofactor can be obtained from Sigma Chemical Company (St. Louis, Missouri).

A diagnostic kit within the present invention may employ volumes of reagents of 10 μl and up. Other types of agglutination slides than that shown in Figure 1 can be used. Also, a cuvette, tube, microtiter plate, or similar device can comprise means for directly visualizing the plasma samples. The method of the present invention can be carried out at a temperature between about 4°C to

50°C, although room temperature is preferred. Once reconstituted, the reagents may be stored from -70°C to 40°C, although higher temperatures tend to shorten the useful life of the reagents.

Fresh-washed platelets, fixed platelets and cryopreserved platelet can be used, so long as ruminant or porcine platelets are employed when a ruminant (or porcine) plasma sample is analyzed. In place of natural platelets, "artificial platelets" can also be used in accordance with the present invention. By "artificial platelets" are meant beads or particles comprised of glass, latex, plastic or other material to which proteins, particlarly platelet-membrane glycoproteins, can be coated and/or surface-bound.  See, e.g., Inman, "Covalent linkage of functional groups, ligands, and proteins to polyacrylaminde beads," 34B METHODS IN ENZYMOLOGY 30 (W.B. Jakoby ed. 1974), the contents of which are hereby incorporated by reference.

For example, the platelet protein glycocalicin, or "glycoprotein I," which is a required component of cofactor-induced factor VIIIR:WF activity, can be isolated, see Solum et al, "Platelet glycocalicin: Its membrane association and solubilization in aqueous media," Biochem. Biophys. Acta 597: 235-246 (1980), and coated onto latex-bead artificial platelets, which in turn can serve as agglutination substrates in the presence of ristocetin or some other cofactor, in the present invention. Alternatively, artificial platelets comprising latex or polyacrylamide beads can be coated with anti-factor VIIIR:Ag antibody, i.e., an antibody which cross-reacts with factor VIIIR:Ag. The artificial platelets, when suspended in a sample comprised of plasma containing the cross-reactive antigen, support a cofactor-induced agglutination which is visualized in accordance with the present invention.

In addition to screening plasma samples for evidence of von Willebrand's disease, the present invention can be used to detect other blood-component deficiencies, including Bernard-Soulier syndrome. It has now been discovered that some horses also suffer a condition substantially similar to Bernard-Soulier syndrome. Both human and equine plasma samples can be screened for Bernard-Soulier syndrome, in accordance with the present invention, by using a platelet-rich plasma, prepared as described in Example 1 above, for the test plasma. If agglutination does not occur after agglutination-inducing cofactor is added to such platelet-rich test plasma, then Bernard-Soulier syndrome can be inferred when an additional test series, using platelet-poor plasma, reconstituted platelets and cofactor as described above, shows normal factor ·VIIIR:WF activity, i.e., when von Willebrand's disease is ruled out as the cause for no agglutination in the platelet-rich test plasma.

These above-described variations, and others, do not constitute a departure from the spirit and scope of the invention, and all such modifications as may be apparent to those of ordinary skill in the art are intended to be included herein.

Claims:

1.   A diagnostic kit for analyzing blood plasma of a particular species, comprising

(a)   first means for visualizing finely-divided punctate bodies dispersed in a predetermined amount of liquid, said first means comprising means for comparing, by direct ocular inspection, at least two samples, each sample comprising a predetermined amount of liquid and at least one sample containing the finely-divided punctate bodies;

(b)   second means for introducing into the first means a predetermined amount of a mixture comprising blood plasma and particles capable of agglutination in the blood plasma in the presence of a cofactor; and

(c)   third means for introducing into the first means a predetermined amount of a solution comprising a cofactor for agglutination of the particles.

2.   A diagnostic kit according to claim 1, wherein said cofactor is selected from ristocetin, ristomycin, a derivative of snake venom capable of inducing agglutination, polybrene, bovine antihemophilic factor and porcine antihemophilic factor.

3.   A diagnostic kit according to claim 1 or 2 wherein said third means comprises (i) means for introducing into said first means a solution comprising a first cofactor for said agglutination and (ii) means for introducing into said first means a solution comprising a second cofactor for said agglutination.

4.   A diagnostic kit according to claim 3, wherein said first cofactor comprises a derivative of snake venom capable of inducing agglutination and said second cofactor comprises ristocetin.

5.   A diagnostic kit according to claim 4, wherein said snake venom is from a snake of the family Crotalidae.

6.   A diagnostic kit according to any of the preceding claims, wherein said second means comprises a plurality of separate means for introducing into said first means, respectively, a predetermined amount of a solution comprising a known concentration of pooled-normal blood plasma, each of said separate means introducing a solution having a differing concentration of said pooled-normal blood plasma, and wherein said second means further comprises means for introducing into said first means a predetermined amount of blood plasma from a particular individual.

7. A diagnostic kit according to claim 6, wherein said second means comprises separate means for introducing into said first means, respectively, (i) a predetermined amount of pooled-normal blood plasma at normal concentration, (ii) a predetermined amount of about 50% normal concentration of said pooled-normal blood plasma, and (iii) a predetermined amount of about 25% normal concentration of said pooled-normal blood plasma.

8. A diagnostic kit according to any of the preceding claims, wherein said mixture further comprises albumin in an amount e.g. about 300 mg per ml, effective to substantially prevent precipitation of plasma proteins by said cofactor during use of said kit.

9. A diagnostic kit according to any of the preceding claims wherein said first means comprises a plurality of wettable substrates, each substrate of said plurality comprising a mirror surface and each of said wettable substrates being adapted to receive a layered sample comprising a predetermined amount of liquid.

10. A diagnostic kit according to any of the preceding claims, wherein said particles comprise blood platelets selected from fresh-washed platelets, fixed platelets, and cryopreserved platelets, or artificial platelets.

11. A diagnostic kit according to claim 10, further comprising a fourth means for reconstituting fresh-washed platelets, fixed platelets, or cryopreserved platelets.

12. A diagnostic kit according to any of the preceding claims wherein said means for comparing said samples comprises a reproduction of an image comprised of punctate bodies having a predetermined density and/or distribution of sizes, or a series of reproductions of images, each image being comprised of punctate bodies having a density and/or a distribution of sizes which varies in a predetermined manner from image to image in said series, and/or said means for comparing said samples comprises means for magnifying the apparent size of said punctate bodies.

13. A diagnostic kit for analysing blood plasma for factor VIII R:WF comprising

(a) agglutination slides or other means for displaying test and control samples in a manner permitting ocular comparison;

(b) lyophilised blood platelets, normal plasma, and at least one dilution of normal plasma, or means for reconstituting said normal and diluted plasma; and

(c) cofactors for agglutination of said platelets, said cofactors comprising (i) a cofactor for agglutination of said platlets in plasma containing normal factor VIIIR:WF, and (ii) a cofactor for agglutination of said platelets in plasma containing abnormal factor VIIIR:WF (i.e. as present in Type II von Willebrand's disease).

14. A diagnostic kit according to claim 13 wherein said cofactor (i) comprises ristocetin and cofactor (ii) comprises a snake venom cofactor.

15. A method for analyzing blood plasma, comprising the steps of

(a) preparing a plurality of samples, each sample having a known volume and comprising, respectively, a predetermined concentration of blood plasma of a first species and particles capable of agglutination in the blood plasma in the presence of a cofactor, the plurality of samples comprising at least (i) a first sample comprising the particles and a known concentration of normal plasma of the first species and (ii) a second sample comprising a known concentration of the particles and blood plasma from a particular individual of the first species;

(b) adding to at least some of the samples of the plurality of samples a predetermined amount of a cofactor for agglutination of the particles in blood plasma of the first species; and then,

(c) after an interval of a duration such that agglutination of said particles by said cofactor is substantially complete, identifying by direct ocular inspection those samples of said plurality of samples wherein punctate bodies are dispersed.

16. A method according to claim 15, wherein said particles comprise blood platelets of a second species, said first species and said second species being the same or different, such that said first species must be a ruminant or porcine if said second species is a ruminant or porcine.

17. A method according to claim 15, wherein said particles comprise artificial platelets.

18. A method according to any of claims 17 to 17 wherein said interval is at least about 3 minutes in duration.

19. A method according to any of claims 15 to 18 wherein step (b) comprises (i) added a first cofactor for agglutination of said particles to a predetermined number of the samples of said plurality of samples, and (ii) adding a second cofactor for agglutination of said particles to at. least some of the remaining samples of said plurality.

20. A method according to any of claims 15 to 19 wherein said plurality of samples comprises (i) a first set of samples, each comprising pooled-normal plasma of said first species, said concentration of blood plasma differing in a predetermined manner from sample to sample in said first set, and (ii) at least one sample comprising a known concentration of blood plasma from a particular individual of said first species.

21. A method according to claim 20, wherein step (b) comprises (i) adding a first cofactor for agglutination of said particles to a predetermined number of samples of said first set and to one sample comprising a known concentration of blood plasma from said particular individual; and (ii) adding a second cofactor for agglutination of said particles to the remaining samples of said first set and to a second sample comprising a known concentration of blood plasma from said particular individual.

22. A method according to claim 19, 20 or 21 wherein said first cofactor comprises ristocetin and said second cofactor comprises a derivative of a snake venom capable of inducing agglutination of said particles.

23. A method according to claim 22, wherein said snake venom is from a snake of the family Crotalidae.

24. A method according to claim 22 or 23 wherein ristocetin is added to at least one sample comprising a known concentration of said pooled-normal plasma and said derivative of said snake venom is added to at least one other sample comprising a known concentration of said pooled-normal plasma.

25. A method according to any of claims 15 to 23 wherein at least said second sample further comprises approximately 300 mg of albumin per ml of said second sample and wherein said first species in canine.

26. A method according to any of claims 15 to 24 wherein said first and said second species are porcine or a ruminant and said cofactor comprises at least one of ristocetin, ristomycin, a derivative of snake venom capable of inducing agglutination, and polybrene.

27. A method according to any of claims 15 to 24 wherein said blood plasma of said first species comprises platetlet-rich blood plasma and wherein said first and second species are equine.

0186476

Fig. 1

Fig. 2
slide

A1
A1
8
10
1
9

1

Fig. 3
5 ml bottle

13
12
11

Fig. 5
bulb
and
tip

6
7

Fig. 4
2 ml bottle

15
6
14

0186476